# FASCICULE DE BREVET EUROPEEN

(11) **EP 3 669 773 B1**
(45) Date de publication et mention de la délivrance du brevet: **05.01.2022**
(21) Numéro de dépôt: 19214173.7
(22) Date de dépôt: 06.12.2019
(51) Int. Cl.: A61B 5/00, A61N 1/39, A61B 5/0245, A61N 1/362

(54) **DISPOSITIF ET PROCÉDÉ DE DÉTECTION DE FIBRILLATION VENTRICULAIRE**
VORRICHTUNG UND VERFAHREN ZUR ERKENNUNG VON HERZKAMMERFLIMMERN
DEVICE AND METHOD FOR DETECTING VENTRICULAR FIBRILLATION

(30) Priorité: 20.12.2018 FR 1873536
(43) Date de publication de la demande: 24.06.2020
(73) Titulaire: Sorin CRM SAS, 92140 Clamart (FR)
(72) Inventeur: CORDERO ALVAREZ, Rafael, 75014 Paris (FR); FEUERSTEIN, Delphine, 92100 Boulogne Billancourt (FR)
(74) Mandataire: Grünecker Patent- und Rechtsanwälte PartG mbB

(56) Documents cités:
- EP-A1- 1 622 679
- EP-A1- 2 364 639
- RAMI J. OWEIS ET AL: "Heart Sounds Segmentation Utilizing Teager Energy Operator", JOURNAL OF MEDICAL IMAGING AND HEALTH INFORMATICS, vol. 4, no. 4, 1 août 2014 (2014-08-01), pages 488-499, XP055642678, ISSN: 2156-7018, DOI: 10.1166/jmihi.2014.1292
- HAMED BEYRAMIENANLOU ET AL: "An Efficient Teager Energy Operator-Based Automated QRS Complex Detection", JOURNAL OF HEALTHCARE ENGINEERING, vol. 2018, 18 septembre 2018 (2018-09-18), pages 1-11, XP055641559, Brentwood ISSN: 2040-2295, DOI: 10.1155/2018/8360475

## Description

La présente invention telle que définie dans les revendications indépendantes 1, 12 et 21 se rapporte à un dispositif médical en particulier à un dispositif médical implantable, ainsi qu'à un procédé et un logiciel pour détecter une fibrillation ventriculaire.

La mort subite d'origine cardiaque, provoquée par une tachyarythmie ventriculaire, reste une des principales causes de décès dans les pays développés. La détection rapide de tels épisodes est d'une importance cruciale pour permettre une réaction immédiate et la délivrance d'un traitement approprié. L'état cardiaque d'un survivant d'une tachyarythmie ventriculaire est étroitement surveillé à l'hôpital, généralement avec un système de surveillance ECG. La surveillance de l'activité cardiaque pour la détection de tachyarythmie ventriculaire implique généralement l'utilisation de signaux électrophysiologiques tels que des électrocardiogrammes (ECG) ou des électrogrammes (EGM). Ces signaux donnent des informations d'origine électrique du fonctionnement du cœur et sont utilisés pour détecter la survenue d'une tachyarythmie ventriculaire telle que la fibrillation ventriculaire.

Alors que le monitorage ECG à l'hôpital constitue une solution viable à court terme, bien que coûteuse, diverses options thérapeutiques à long terme peuvent être recommandées pour les patients présentant un risque cardiaque de mort subite. Celles-ci comprennent un traitement pharmacologique et/ou l'implantation d'un défibrillateur. Les défibrillateurs automatiques implantables comprennent des électrodes qui enregistrent les EGM. Les défibrillateurs automatiques implantables sous-cutanés sont capables d'enregistrer des ECG. Ces EGM/ECG sont traités par des algorithmes dans le dispositif afin de détecter un rythme cardiaque qui peut indiquer, par exemple, la présence d'une fibrillation ventriculaire. Néanmoins, ces algorithmes peuvent donner lieu à des faux positifs, souvent causés par d'autres sources de bruit électrophysiologique. Les faux positifs correspondent à la détection de phénomène tel que le bruit électrique, les myopotentiels, la double détection d'un QRS large, la détection d'ondes T, la tachycardie supraventriculaire ou tout autre phénomène électrophysiologique qui ne correspond pas à la tachyarythmie ventriculaire mais qui est détecté par erreur en tant que telle. En effet, la présence fréquente de bruit de fond électrophysiologique réduit le rapport signal/bruit. Dans un défibrillateur automatique implantable, par exemple, un faux positif entraînerait un choc inapproprié, pouvant être traumatique, voire délétère par l'induction d'une tachyarythmie ventriculaire.

La demande US 2004/225332 A1 concerne un système et procédé de détection et de discrimination des arythmies cardiaques sur la base de signaux mécaniques du mouvement de la paroi cardiaque et de signaux électriques de dépolarisation cardiaque. L'algorithme de US 2004/225332 A1 compare et corrobore les signaux mécaniques détectés avec les signaux électriques détectés afin de classifier le rythme cardiaque. Ainsi, US 2004/225332 A1 utilise, en addition à des signaux électrophysiologiques, des signaux d'activité mécaniques cardiaques pour la détection d'arythmie ventriculaire- qui est normalement détectée seulement à partir de signaux électrophysiologiques. L'algorithme de US 2004/225332 A1 n'est cependant pas adapté pour améliorer la détection de fibrillation ventriculaire car il est toujours basé sur des signaux électrophysiologiques qui donnent souvent lieu à des faux positifs du fait de la présence de bruit électrophysiologique provenant d'autres sources (par exemple dus à des myopotentiels).

La demande US 2011/0098587 A1 concerne l'utilisation de capteurs non-électrophysiologiques tels que des accéléromètres ou des transducteurs acoustiques, en plus d'une série d'électrodes qui détectent des signaux électrophysiologiques, qui sont implantés par voie sous-cutanée afin, entre autres, de détecter une tachyarythmie ventriculaire. Cependant, selon la demande US 2011/0098587 A1, les signaux enregistrés qui sont non-électrophysiologiques (par un accéléromètre sous-cutané par exemple) sont utilisés pour sélectionner le meilleur signal de sortie possible d'une série de signaux électrophysiologiques par un processus de « séparation aveugle de source » (en anglais BSS pour « Blind signal séparation » ou « Blind source séparation »). C'est ensuite ce signal de sortie résultant de la séparation aveugle de source qui est utilisé pour classifier les rythmes cardiaques et détecter, par exemple, les tachyarythmies ventriculaires. De ce fait, le procédé de US 2011/0098587 A1 dépend des signaux électrophysiologiques pour la classification des rythmes cardiaques. Or, de tels signaux, s'ils sont pollués par du bruit, tels que des myopotentiels qui ne sont pas supprimés au cours du processus de séparation, peuvent entraîner la détection de faux positifs. Ainsi, le dispositif de US 2011/0098587 A1 n'est pas adapté pour améliorer la détection de fibrillation ventriculaire. Le document "Heart Sounds Segmentation Utilizing Teager Energy Operator" to RAMI J. OWEIS et al. in Journal of medical imaging and health informatics, vol. 4, N° 4, 01-08-2014, p. 488-499, décrit l'utilisation d'un opérateur TEO pour la détection des différents complexes S1, S2, S3, S4 dans des signaux sonores du coeur.

La présente invention a pour objet d'améliorer la spécificité de la discrimination d'un événement cardiaque, en particulier de fibrillation ventriculaire, et de réduire l'occurrence de résultats de détection faux positifs, c'est-à-dire la détection de phénomène tel que le bruit électrique, les myopotentiels, la double détection d'un QRS large, la détection d'ondes T, la tachycardie supraventriculaire ou tout autre phénomène électrophysiologique qui ne correspond pas à la tachyarythmie ventriculaire mais qui est détecté par erreur en tant que telle.

L'objet de la présente invention est atteint par un dispositif médical, en particulier un dispositif médical implantable, comprenant au moins un capteur hémodynamique implantable ou non-implantable configuré pour détecter des signaux hémodynamiques cardiaques; un contrôleur configuré pour traiter et analyser les signaux hémodynamiques cardiaques détectés et/ou de signaux dérivés des signaux hémodynamiques cardiaques détectés en appliquant auxdits signaux un Opérateur d'Energie Teager (TEO) défini par: TEO {x (n)} =Ψ (n) = x².(n-1)-x(n-2).x(n) où « x (n) » est un signal hémodynamique cardiaque, « Ψ (n) » est le signal hémodynamique de sortie et « n » fait référence à un échantillon prédéterminé ; ledit contrôleur comprenant en outre au moins un algorithme configuré pour déterminer la nécessité d'une opération de défibrillation en prenant en compte le au moins un signal hémodynamique de sortie.

L'énergie mécanique générée par le cœur pendant une fibrillation ventriculaire est grandement réduite par rapport à celle d'un rythme cardiaque sinusal normal. Le dispositif de l'invention utilise ce fait pour détecter les épisodes de fibrillation ventriculaire en fonction du ou des signaux hémodynamiques cardiaques détectés ou de leurs signaux dérivés. La transformation par application de l'opérateur TEO permet d'augmenter le rapport signal/bruit des signaux de sortie et procure ainsi une représentation des signaux détectés ou des signaux dérivés des signaux détectés, à partir de laquelle il est plus facile de déterminer si des sons cardiaques sont présents ou non (c'est-à-dire s'il existe ou non une activité cardiaque mécanique). En effet, le traitement des signaux par l'opérateur TEO permet de condenser le son du cœur essentiellement en un seul pic.

A propos des résultats faux-positifs, le dispositif de l'invention est capable de s'acquitter des signaux électrophysiologiques relatifs à une activité électrique pour détecter une fibrillation ventriculaire, tels que des électrocardiogrammes (ECG) ou des électrogrammes (EGM) qui sont usuellement utilisés pour détecter une tachyarythmie, mais qui ont tendance à engendrer des résultats de détection faux positifs, surtout dans le cas d'une fibrillation ventriculaire. Ainsi, grâce à la représentation TEO, la détection des épisodes de fibrillation ventriculaire peut être effectuée plus facilement tout en réduisant l'occurrence de résultats de détection faux positifs.

La présente invention se rapportant à un dispositif médical peut être davantage améliorée grâce aux modes de réalisation suivants.

Selon un mode de réalisation, le au moins un capteur hémodynamique peut être un accéléromètre implantable ou non-implantable, un microphone, un capteur piézoélectrique ou un capteur de pression apte à détecter des signaux cardiaques hémodynamiques, en particulier des sons cardiaques. Un accéléromètre est configuré pour détecter des signaux cardiaques hémodynamiques et peut être implanté soit dans le cœur, soit par voie sous-cutanée, soit attaché de manière externe sur la peau. Les capteurs hémodynamiques pouvant être utilisés pour la mise en oeuvre du dispositif selon la présente invention peuvent ainsi être implantables, non-implantables (sous-cutanés ou cutanés). De ce fait, les capteurs hémodynamiques peuvent être localisés à différents endroits dans ou/et sur le corps du patient.

Selon un mode de réalisation, le au moins un algorithme du contrôleur peut être configuré pour déterminer la nécessité d'une opération de défibrillation si au moins une caractéristique des signaux hémodynamiques de sortie ne franchit pas un seuil prédéterminé pendant un lapse de temps qui est supérieur à une durée associée à un épisode de fibrillation ventriculaire. De plus, la au moins une caractéristique du signal hémodynamique de sortie peut être l'amplitude, l'énergie, la valeur moyenne ou la valeur médiane, ou la valeur quadratique moyenne dudit signal. L'invention repose ainsi sur l'absence d'activité plutôt que sur la présence d'une activité accrue où des artefacts de bruit électrophysiologiques risqueraient d'affecter le signal d'activité mécanique cardiaque. De plus, même si des artefacts mécaniques analogues étaient présents dans le signal, ils ne donneraient pas de faux positifs.

Selon un mode de réalisation, l'amplitude des signaux hémodynamiques cardiaques de sortie, et/ou le nombre de fois que chacun des signaux hémodynamiques cardiaques de sortie franchit de manière ascendante ou descendante le seuil prédéterminé, et/ou le nombre de pics de chaque signal qui dépasse le seuil prédéterminé pendant le lapse de temps prédéfini, peut/peuvent être pris(e) en compte pour déterminer la présence ou non d'une fibrillation ventriculaire. Le traitement TEO ayant permis d'améliorer la netteté des signaux, et donc des pics, la détermination d'une fibrillation ventriculaire par rapport à un seuil est facilitée.

Selon un mode de réalisation, le dispositif médical peut en outre comprendre un défibrillateur, en particulier un défibrillateur implantable, configuré pour générer un signal de défibrillation électrique ; et au moins une électrode connectée audit défibrillateur par une sonde configurée pour délivrer ledit signal de défibrillation électrique à un patient lorsque la nécessité de déclencher une opération défibrillation est déterminée au moyen du contrôleur du dispositif médical. Ainsi, le dispositif est également configuré pour traiter une fibrillation ventriculaire, suite à la détection d'un épisode de fibrillation ventriculaire, au moyen d'un défibrillateur.

Selon un mode de réalisation, le contrôleur peut être configuré pour déterminer la nécessité de déclencher une opération de défibrillation en comparant les signaux hémodynamiques cardiaques de sortie à au moins un signal électrophysiologique détecté de manière indépendante aux signaux hémodynamiques cardiaques au moyen de la au moins une électrode. Le fait de prendre en compte et comparer des signaux de natures différentes (électrophysiologique et hémodynamiques) qui sont détectés de manière indépendante l'un de l'autre permet de vérifier et d'améliorer la fiabilité du résultat (présence ou non d'un épisode de fibrillation ventriculaire).

Selon un mode de réalisation, le contrôleur peut être configuré pour déterminer la nécessité d'une opération de défibrillation en prenant en compte uniquement des signaux hémodynamiques cardiaques. Le dispositif peut ainsi s'affranchir de la détection et du traitement de signaux électrophysiologiques, ce qui simplifie la détection de fibrillation ventriculaire.

Selon un mode de réalisation, le contrôleur peut être configuré pour filtrer par passe-bande les signaux hémodynamiques cardiaques détectés et/ou des signaux dérivés des signaux hémodynamiques cardiaques détectés dans une gamme de 1 à 100 Hz, en particulier de 7,5 à 49 Hz. Le filtrage des signaux hémodynamiques dans cette étroite bande de fréquences permet d'éliminer les contributions respiratoires à basse fréquence ainsi que les contributions des ondes cardiaques acoustiques à haute fréquence. En outre, cela limite l'influence que les artefacts de mouvement pourraient avoir sur les signaux cardiaques. Ceci est particulièrement pertinent lorsque le patient implanté avec un tel dispositif bouge et, par exemple, fait de l'exercice physique.

Selon un mode de réalisation, le contrôleur peut en outre être configuré pour filtrer les signaux de sortie par application d'une fonction de fenêtrage, en particulier une fenêtre de Hamming. La fenêtre de Hamming, qui a une forme sinusoïdale, permet de rendre les pics des signaux de sortie plus grands par rapport au bruit mais aussi de faciliter le traitement et l'analyse des signaux de sortie, car elle réduit le risque de doubles détections (c'est-à-dire plusieurs pics situés à proximité les uns des autres et pouvant être à tort assimilés à un seul pic).

Selon un mode de réalisation, le contrôleur peut être configuré pour déterminer la nécessité d'une opération de défibrillation et/ou déclencher une opération de défibrillation selon un seuil statique prédéfini, ou selon un seuil dynamique, recalculé périodiquement et dérivé d'une ou de plusieurs caractéristiques des signaux hémodynamiques cardiaques ou de leur représentation par l'Opérateur d'Energie Teager. Ainsi, la valeur du seuil prédéterminé peut être une valeur constante qui est définie a priori. Alternativement, le seuil dynamique utilisé pour établir la présence ou non d'une fibrillation ventriculaire est davantage spécifique et permet ainsi d'affiner la détection de l'épisode de fibrillation ventriculaire.

Les modes de réalisation peuvent être combinés pour former davantage de variantes de mode de réalisation avantageux de la présente invention.

L'objet de la présente invention peut également être atteint avec un procédé pour traiter de signaux cardiaques hémodynamiques détectés par au moins un capteur hémodynamique d'un dispositif médical, en particulier d'un dispositif médical implantable, comprenant au moins une étape de traitement des signaux hémodynamiques détectés et/ou de signaux dérivés des signaux hémodynamiques cardiaques détectés qui comprend l'application d'un Opérateur d'Energie Teager (TEO) défini par: TEO {x(n)} =Ψ (n) = x².(n-1)-x(n-2).x(n) où « x (n) » est un signal hémodynamique cardiaque détecté, « Ψ (n) » est le signal cardiaque hémodynamique de sortie et « n » fait référence à un échantillon prédéterminé ; et comprenant en outre une étape au cours de laquelle la présence ou non d'une fibrillation ventriculaire est déterminée en prenant en compte le ou les signaux hémodynamiques cardiaques de sortie par rapport à un seuil prédéterminé.

L'énergie mécanique générée par le cœur pendant une fibrillation ventriculaire est grandement réduite par rapport à celle d'un rythme cardiaque sinusal normal. Le procédé de l'invention utilise ce fait pour détecter les épisodes de fibrillation ventriculaire en fonction du ou des signaux hémodynamiques cardiaques détectés ou de leurs signaux dérivés. La transformation par application de l'opérateur TEO permet d'augmenter le rapport signal/bruit des signaux de sortie et procure ainsi une représentation des signaux détectés ou des signaux dérivés des signaux détectés, à partir de laquelle il est plus facile de déterminer si des sons cardiaques sont présents ou non (c'est-à-dire s'il existe ou non une activité cardiaque mécanique).

A propos des résultats faux-positifs, le procédé de l'invention est capable de s'acquitter des signaux électrophysiologiques relatifs à une activité électrique pour arriver à la détection d'une fibrillation ventriculaire, tels que des électrocardiogrammes (ECG) ou des électrogrammes (EGM) qui sont usuellement utilisés pour détecter une tachyarythmie, mais peuvent engendrer des résultats de détection faux positifs, surtout dans le cas d'une fibrillation ventriculaire. Ainsi, grâce à la représentation TEO, la détection des épisodes de fibrillation ventriculaire peut être effectuée plus facilement tout en réduisant l'occurrence de résultats de détection faux positifs.

La présente invention se rapportant à un procédé peut être davantage améliorée grâce aux modes de réalisation suivants.

Selon un mode de réalisation, l'étape de traitement des signaux peut être précédée d'une étape de prétraitement des signaux à laquelle les signaux hémodynamiques cardiaques détectés et/ou les signaux dérivés des signaux hémodynamiques cardiaques détectés sont filtrés par passe-bande, en particulier dans une gamme de 1 à 100 Hz, plus en particulier de 7,5 à 49 Hz. Le filtrage des signaux hémodynamiques dans cette étroite bande de fréquences permet d'éliminer les contributions respiratoires à basse fréquence ainsi que les contributions des ondes cardiaques acoustiques à haute fréquence. En outre, cela limite l'influence que les artefacts de mouvement pourraient avoir sur les signaux cardiaques. Ceci est particulièrement pertinent lorsque le patient équipé et/ou implanté avec un tel dispositif bouge et, par exemple, fait de l'exercice physique.

Selon un mode de réalisation, l'étape de traitement des signaux peut être suivie d'une étape de post-traitement des signaux à laquelle les signaux hémodynamiques de sortie sont filtrés par application d'une fonction de fenêtrage, en particulier par application d'une fenêtre de Hamming. La fenêtre de Hamming permet de rendre les pics des signaux de sortie plus grands par rapport au bruit mais aussi de faciliter le traitement et l'analyse des signaux de sortie, car elle réduit le risque de doubles détections (c'est-à-dire plusieurs pics situés à proximité les uns des autres et pouvant être à tort assimilés à un seul pic).

Selon un mode de réalisation, le au moins un capteur hémodynamique peut être un accéléromètre à N-axes (N >= 1) implantable ou non-implantable, un microphone, un capteur piézoélectrique ou un capteur de pression apte à détecter des signaux cardiaques hémodynamiques, en particulier des sons cardiaques. Un accéléromètre est configuré pour détecter des signaux cardiaques hémodynamiques et peut être implanté soit dans le cœur, soit par voie sous-cutanée, soit attaché de manière externe sur la peau.

Selon un mode de réalisation, les N signaux cardiaques détectés par l'accéléromètre à N-axes peuvent être combinés dans un nouveau signal selon un axe N+1, l'axe N+1 étant déterminé de sorte que l'amplitude et/ou le rapport signal sur bruit et/ou la stabilité et/ou un paramètre physiologique pertinent du nouveau signal est maximale selon l'axe N+1. De cette manière, les signaux peuvent être combinés pour produire un signal différent qui soit plus spécifique au patient et/ou qui maximise les propriétés du signal hémodynamique détecté, telles que l'amplitude, le rapport signal/bruit et/ou la stabilité.

Selon un mode de réalisation, le seuil prédéterminé peut être un seuil statique prédéfini, ou un seuil dynamique, recalculé périodiquement et dérivé d'une ou de plusieurs caractéristiques des signaux hémodynamiques cardiaques ou de leur représentation par l'Opérateur d'Energie Teager. Ainsi, la valeur du seuil prédéterminé peut être une valeur constante et qui est définie a priori. Alternativement, le seuil dynamique utilisé pour établir la présence ou non d'une fibrillation ventriculaire est davantage spécifique et permet ainsi d'affiner la détection de l'épisode de fibrillation ventriculaire.

Selon un mode de réalisation, l'amplitude, et/ou le nombre de fois que chacun des signaux hémodynamiques de sortie franchit de manière ascendante ou descendante le seuil prédéterminé, et/ou le nombre de pics de chaque signal qui dépasse le seuil prédéterminé pendant un lapse de temps prédéfini, peut/peuvent être pris(e) en compte pour déterminer la présence ou non d'une fibrillation ventriculaire. La détermination d'une fibrillation ventriculaire par rapport à un seuil est ainsi facilitée car le traitement TEO a permis d'améliorer la netteté des pics, et donc des signaux.

Selon un mode de réalisation, la présence ou non d'une fibrillation ventriculaire peut être déterminée en prenant en compte uniquement des signaux hémodynamiques cardiaques. Le procédé peut ainsi s'affranchir de la détection, du traitement et de la comparaison avec des signaux électrophysiologiques, ce qui simplifie le procédé permettant la détection de fibrillation ventriculaire.

Alternativement, la présence ou non d'une fibrillation ventriculaire peut être établie en comparant le signal hémodynamique de sortie à au moins un signal électrophysiologique détecté au moyen d'une électrode du dispositif médical. Le fait de prendre en compte et comparer des signaux de natures différentes (électrophysiologique et hémodynamiques) permet de vérifier et d'améliorer la fiabilité du résultat (présence ou non d'un épisode de fibrillation ventriculaire).

L'objet de la présente invention peut également être atteint avec un logiciel de traitement de signaux hémodynamiques cardiaques détectés par au moins un capteur hémodynamique d'un dispositif médical, en particulier d'un dispositif médical implantable, caractérisé en ce qu'il comprend des instructions aptes, lorsqu'elles sont exécutées par le dispositif médical, à exécuter le traitement des signaux hémodynamiques détectés et/ou des signaux dérivés des signaux hémodynamiques cardiaques détectés par application d'un Opérateur d'Energie Teager (TEO) défini par: TEO {x (n)} =Ψ (n) = x².(n-1)-x.(n-2).x(n) où «x(n)» est le signal hémodynamique cardiaque détecté, «Ψ (n)» est le signal de sortie et «n» fait référence à un échantillon prédéterminé ; et à comparer au moins une caractéristique du signal de sortie à un seuil prédéterminé ; et à déclencher une alerte lorsque la au moins une caractéristique du signal de sortie ne franchit pas le seuil prédéterminé pendant un lapse de temps prédéfini.

L'énergie mécanique générée par le cœur pendant une fibrillation ventriculaire est grandement réduite par rapport à celle d'un rythme cardiaque sinusal normal. Le logiciel de l'invention se base sur ce fait pour détecter les épisodes de fibrillation ventriculaire en fonction du ou des signaux hémodynamiques cardiaques détectés ou de leurs signaux dérivés. La transformation par l'opérateur TEO permet d'augmenter le rapport signal/bruit des signaux de sortie et procure ainsi une représentation des signaux originaux à partir de laquelle il est plus facile de déterminer si des sons cardiaques sont présents ou non (c'est-à-dire s'il existe ou non une activité cardiaque mécanique).

De plus, contrairement à d'autres méthodes et algorithmes, tel que le filtrage par ondelettes, l'opérateur TEO ne nécessite pas d'information a priori relative au signal ni un apprentissage pour l'algorithme.

La présente invention se rapportant à un logiciel peut être davantage améliorée grâce aux modes de réalisation suivants.

Selon un mode de réalisation, le logiciel peut en outre comprendre le prétraitement des signaux hémodynamiques cardiaques détectés et/ou des signaux dérivés des signaux hémodynamiques cardiaques détectés par filtrage passe-bande, en particulier dans une gamme de 1 à 100 Hz, plus en particulier de 7,5 à 49 Hz. Le pré-filtrage des signaux hémodynamiques dans cette étroite bande de fréquences permet d'éliminer les contributions respiratoires à basse fréquence ainsi que les contributions des ondes cardiaques acoustiques à haute fréquence. En outre, cela limite l'influence que les artefacts de mouvement pourraient avoir sur les signaux cardiaques. Ceci est particulièrement pertinent lorsque le patient implanté avec un tel dispositif bouge et, par exemple, fait de l'exercice physique.

Selon un mode de réalisation, le logiciel peut en outre comprendre le post-traitement du signal de sortie par application d'une fonction de fenêtrage, en particulier une fenêtre de Hamming. La fenêtre de Hamming permet de rendre les pics des signaux de sortie plus grands par rapport au bruit mais aussi de faciliter le traitement et l'analyse des signaux de sortie, car elle réduit le risque de doubles détections (c'est-à-dire plusieurs pics situés à proximité les uns des autres et pouvant être à tort assimilés à un seul pic).

L'invention et ses avantages seront expliqués plus en détail dans la suite au moyen de modes de réalisation préférés et en s'appuyant notamment sur les figures d'accompagnement suivantes, dans lesquelles :
Figure 1a représente un dispositif médical comprenant un capteur hémodynamique cardiaque selon un premier mode de réalisation de l'invention.
Figure 1b représente un dispositif médical comprenant un capteur hémodynamique cardiaque selon un deuxième mode de réalisation de l'invention.
Figure 1c représente un dispositif médical comprenant un capteur hémodynamique cardiaque selon un troisième mode de réalisation de l'invention.
Figure 1d représente un ensemble comprenant un capteur hémodynamique cardiaque selon un quatrième mode de réalisation de l'invention.
Figure 2 représente des signaux hémodynamiques durant une fibrillation ventriculaire.
Figure 3 représente un accéléromètre à trois axes.
Figure 4a représente un algorithme selon un premier mode de réalisation de l'invention.
Figure 4b représente un algorithme selon un deuxième mode de réalisation de l'invention.
Figure 4c représente un algorithme selon un troisième mode de réalisation de l'invention.
Figure 4d représente un algorithme selon un quatrième mode de réalisation de l'invention.

L'invention va maintenant être décrite plus en détail en utilisant des modes de réalisation avantageux d'une manière exemplaire et en référence aux dessins. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

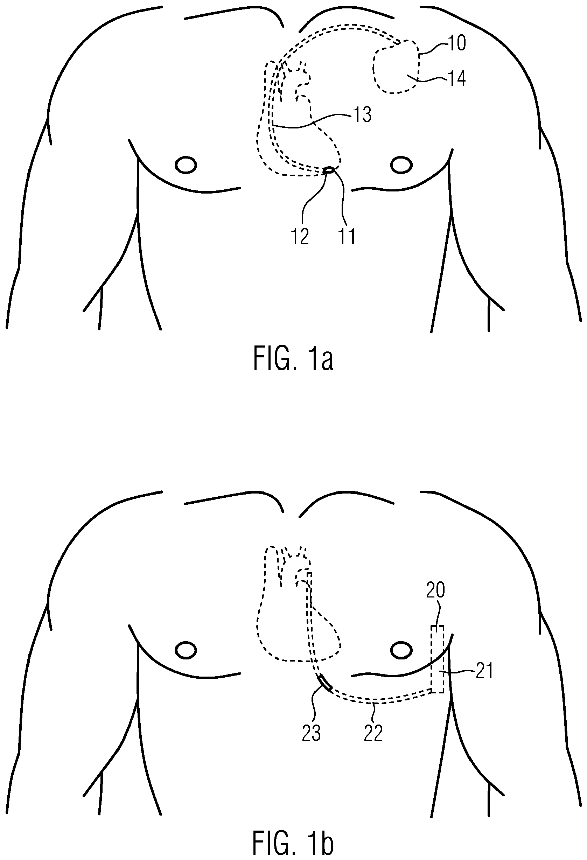

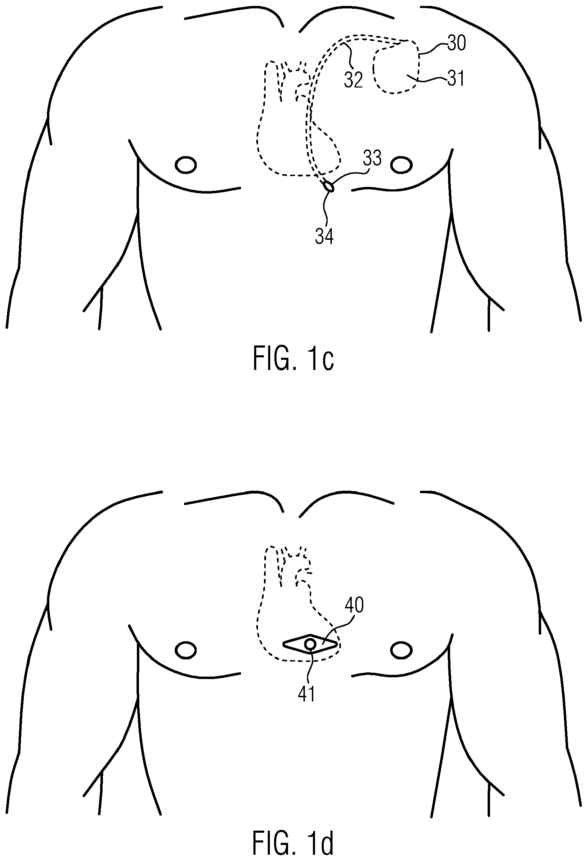

Selon la présente invention, les signaux hémodynamiques sont enregistrés par au moins un capteur hémodynamique cardiaque, tel un accéléromètre qui peut être intégré à l'intérieur d'un boîtier d'un défibrillateur, notamment d'un défibrillateur automatique implantable ou dans une sonde connectée au défibrillateur selon divers modes de réalisations (voir Figures 1a-1c). Selon un mode de réalisation alternatif, le capteur hémodynamique cardiaque peut être distinct d'un défibrillateur, c'est-à-dire non-associé, non-intégré ou non-combiné à un défibrillateur (voir Figure 1d).

La **Figure** 1a illustre un dispositif médical 10, en particulier un défibrillateur automatique implantable 10 selon un premier mode de réalisation, qui est implanté par voie sous-cutanée avec un accéléromètre 11 mono- ou multi-axe(s) intégré à l'extrémité distale 12 de la sonde de stimulation intracardiaque 13 du défibrillateur 10. La sonde de stimulation intracardiaque 13 peut également comporter une ou plusieurs électrodes (non-représentées) pour enregistrer des signaux électrophysiologiques. Le défibrillateur automatique implantable 10 qui est connecté à la sonde de stimulation intracardiaque 13, est conçu pour loger dans un boîtier 14 un microcontrôleur et des circuits électroniques associés (non-représentés).

Le dispositif médical 10 comprend un contrôleur (non représenté) ayant un algorithme configuré pour détecter une fibrillation ventriculaire en prenant en compte les signaux hémodynamiques cardiaques détectés par l'accéléromètre 11. Selon la présente invention, les signaux électrophysiologiques détectés par la ou les électrodes de la sonde de stimulation intracardiaque 13 ne sont pas utilisés par ledit algorithme du contrôleur. En effet, le contrôleur du dispositif médical 10 est apte à détecter un épisode de fibrillation ventriculaire en prenant uniquement en compte les signaux hémodynamiques.

De plus, selon le premier mode de réalisation de l'invention, le contrôleur du dispositif médical 10 comprend également un algorithme configuré pour déclencher une opération de défibrillation en prenant en compte les signaux hémodynamiques. Ainsi, le dispositif 10 est également configuré pour traiter une fibrillation ventriculaire, suite à la détection d'un épisode de fibrillation ventriculaire, au moyen d'un signal de défibrillation délivrée par le défibrillateur 10, notamment par la sonde de stimulation intracardiaque 13 du défibrillateur 10.

La **Figure 1b** illustre un dispositif médical 20, en particulier un défibrillateur automatique implantable 20 selon un deuxième mode de réalisation, dont le boîtier 21 est implanté de manière sous-cutanée sous l'aisselle d'un patient. Le boîtier 21 du dispositif 20 comprend un microcontrôleur et des circuits électroniques associés (non-représentés) et est connecté à une sonde 22 sous-cutanée dans la région parasternale comportant elle-même une ou plusieurs électrodes (non-représentées). Des capteurs hémodynamiques cardiaques, tel qu'un accéléromètre 23, peuvent être intégrés au boîtier 21 du dispositif 20 et/ou à la sonde 22 sous-cutanée.

Le dispositif médical 20 comprend un contrôleur (non représenté) ayant un algorithme configuré pour détecter une fibrillation ventriculaire en prenant en compte les signaux hémodynamiques cardiaques détectés par l'accéléromètre 23. Selon la présente invention, les signaux électrophysiologiques détectés par la ou les électrodes de la sonde 22 ne sont pas utilisés par ledit algorithme du contrôleur. En effet, le contrôleur du dispositif médical 20 est apte à détecter un épisode de fibrillation ventriculaire en prenant uniquement en compte les signaux hémodynamiques.

De plus, selon le deuxième mode de réalisation de l'invention, le contrôleur du dispositif médical 20 comprend également un algorithme configuré pour déclencher une opération de défibrillation en prenant en compte les signaux hémodynamiques. Ainsi, le dispositif 20 est également configuré pour traiter une fibrillation ventriculaire, suite à la détection d'un épisode de fibrillation ventriculaire, au moyen d'un signal de défibrillation délivrée par le défibrillateur 20.

De la même manière, la **Figure 1c****,** illustre un dispositif médical 30, en particulier un défibrillateur automatique implantable 30 selon un troisième mode de réalisation, dont le boîtier 31 est implanté de manière sous-cutanée dans la poche pectorale. Le boîtier 31 du défibrillateur 30 comprend un microcontrôleur et des circuits électroniques associés (non-représentés). Le boîtier 31 connecté à une sonde de stimulation 32 sous-cutanée dans la région parasternale comportant elle-même une ou plusieurs électrodes (non-représentées). Un capteur hémodynamique cardiaque, ici un accéléromètre 33, est intégré à l'extrémité distale 34 de la sonde de stimulation 32.

Le dispositif médical 30 comprend un contrôleur (non représenté) ayant un algorithme configuré pour détecter une fibrillation ventriculaire en prenant en compte les signaux hémodynamiques cardiaques détectés par l'accéléromètre 33. Selon le troisième mode de réalisation de la présente invention, les signaux électrophysiologiques détectés par la ou les électrodes de la sonde de stimulation 32 ne sont pas utilisés par ledit algorithme du contrôleur. En effet, le contrôleur du dispositif médical 30 est apte à détecter un épisode de fibrillation ventriculaire en prenant uniquement en compte les signaux hémodynamiques.

Selon un mode de réalisation alternatif, les signaux électrophysiologiques qui sont détectés de manière indépendante aux signaux cardiaques hémodynamiques sont pris en compte par le contrôleur de manière à comparer les deux types de signaux (électrophysiologique et hémodynamique) entre eux afin d'améliorer la fiabilité de la détection d'un épisode de fibrillation ventriculaire.

De plus, selon le troisième mode de réalisation de l'invention, le contrôleur du dispositif médical 30 comprend également un algorithme configuré pour déclencher une opération de défibrillation en prenant en compte les signaux hémodynamiques. Ainsi, le dispositif 30 est également configuré pour traiter une fibrillation ventriculaire, suite à la détection d'un épisode de fibrillation ventriculaire, au moyen d'un signal de défibrillation délivrée par le défibrillateur 30, notamment de la sonde de stimulation 32.

La **Figure 1d****,** illustre un quatrième mode de réalisation dans lequel un capteur hémodynamique cardiaque 40, en particulier un accéléromètre, est incorporé au centre d'un pansement ou d'un bandage 41 collé sur la poitrine d'un patient. Ce pansement ou ce bandage 41 peut aussi comporter en outre une ou plusieurs électrodes (non-représentées), un microcontrôleur et des circuits électroniques associés (non-représentés). Dans ce quatrième mode de réalisation, lorsqu'une fibrillation ventriculaire est détectée au moyen du capteur hémodynamique cardiaque 40 du bandage 41, un défibrillateur externe (automatique ou manuel) peut être utilisé pour une opération de défibrillation. Selon une variante, le capteur hémodynamique cardiaque 40 est à la fois distinct d'un défibrillateur (c'est-à-dire, par exemple, qu'il n'est pas intégré ou connecté à un défibrillateur et/ou à une de ses sondes de stimulation) et n'est pas utilisé en combinaison avec un défibrillateur.

Ainsi, selon les différents modes de réalisation de l'invention, les signaux hémodynamiques sont détectés au moyen d'au moins un capteur hémodynamique, tel qu'un accéléromètre 11, 23, 33, 40 qui est soit implanté à l'intérieur du cœur 11, soit sous-cutanée 23, 33 ou fixé sur l'épiderme de la poitrine d'un patient 40.

Dans un autre mode de réalisation, le capteur hémodynamique peut être un microphone, un capteur piézoélectrique, un capteur de pression ou similaire.

De plus, selon une variante de l'invention, l'enregistrement de signaux hémodynamiques peut être réalisé à partir d'une combinaison de plusieurs capteurs hémodynamiques de même types (par exemple plusieurs accéléromètres) ou de catégories différentes (par exemple un capteur implanté et un capteur cutané) qui seraient positionnés à divers endroits du corps d'un patient.

Selon le mode de réalisation de l'invention, chacun des capteurs hémodynamiques peut être utilisé, ou non, en combinaison avec un défibrillateur, en particulier un défibrillateur implantable.

Quel que soit le mode de réalisation de l'invention, la présente invention prend en compte l'énergie nécessaire à la génération d'un signal hémodynamique cardiaque, tel que celui enregistré par un accéléromètre, afin de transformer le signal en représentation alternative facilitant considérablement la détection de fibrillation ventriculaire. Cette représentation de l'énergie est fournie par l'application de TEO (pour « Teager Energy Operator » en anglais) sur le signal hémodynamique cardiaque.

L'opérateur Teager Energy (TEO dans la suite) est défini par l'équation suivante Ψ(n) = x².(n-1)-x(n-2).x(n) où x (n) est le signal d'entrée (dans ce cas, le signal hémodynamique cardiaque) et Ψ (n) est le signal de sortie de l'opérateur. "n" désigne un échantillon particulier.

Le TEO opérateur (« Teager Energy Operator » en anglais) est un opérateur mathématique qui peut être intégré au logiciel ou au hardware d'un dispositif cardiaque implantable, tel qu'un défibrillateur automatique implantable, utilisant des capteurs hémodynamiques, tels que des accéléromètres, pour enregistrer les signaux hémodynamiques cardiaques. Les capteurs peuvent être intracardiaques, sous-cutanés ou externes (c'est-à-dire avec des capteurs fixés sur la peau du patient).

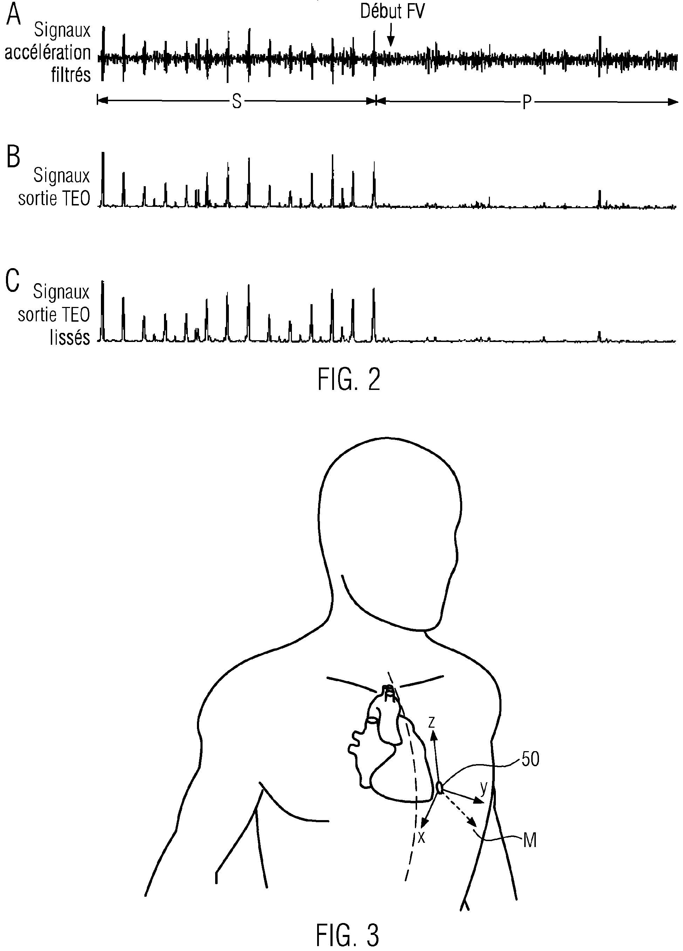

La **Figure 2** représente graphiquement quelques secondes d'un signal du rythme sinusal normal avant (S) et pendant (P) un épisode de fibrillation ventriculaire. Le graphique A illustre le signal acquis par l'accéléromètre (A) et filtré, le graphique B illustre le signal de sortie TEO et le graphique C illustre le signal de sortie TEO lissé.

La transformation d'un signal par l'opérateur TEO (pour « Teager Energy Operator » en anglais) produit une estimation en temps réel de l'énergie nécessaire qu'il a fallu pour générer le signal d'entrée. Pendant un rythme sinusal normal, la sortie TEO d'un signal d'un accéléromètre hémodynamique cardiaque tel que décrit aux Figures 1a-d apparaît sous la forme d'une série de pics distincts et nets qui coïncident avec les sons du cœur, comme illustré par le graphique A de la Figure 2. De manière générale, le premier son du cœur, communément appelé son S1 a une amplitude plus grande que second son du cœur, ou son S2. De ce fait, le pic du signal de sortie TEO correspondant au premier son cardiaque S1 est nettement supérieur à celui du second S2. Entre les sons S1, S2 du cœur, le signal de sortie TEO est plat comme illustré par les graphiques B et C de la Figure 2. Cela est dû au fait que le bruit de fond est généralement d'amplitude et de fréquence plus faibles par rapport aux sons S1, S2 du cœur. En conséquence, la transformation d'un signal avec l'opérateur TEO permet d'améliorer considérablement le rapport signal/bruit du signal où ledit signal du rapport signal/bruit correspond au son S1 du cœur (provoqué par la turbulence causée par la fermeture des valves mitrale et tricuspide au début de la systole) tandis que le reste du signal correspond au bruit. De ce fait, la présente invention permet d'améliorer la spécificité de la discrimination d'un événement cardiaque, en particulier de fibrillation ventriculaire.

En effet, au cours d'une fibrillation ventriculaire, la contraction synchronisée et coordonnée des ventricules est remplacée par un tremblement désorganisé ou anarchique. Les performances mécaniques cardiaques sont gravement altérées et les ventricules ne se contractent pas avec la même énergie que lors d'un fonctionnement normal. Ceci est reflété dans le signal de sortie TEO d'un signal hémodynamique cardiaque sous la forme d'un plateau plat prolongé proche de zéro et représenté par la double flèche P sur la Figure 2. Les pics caractéristiques de grande amplitude reflétant les sons cardiaques S1, S2 (voir double flèche S) sont absents.

Tel qu'illustré par le graphique A de la Figure 2, les signaux enregistrés avec un capteur hémodynamique cardiaque tel qu'un accéléromètre mono- ou multi-axe(s), c'est-à-dire à N-axe (où N ≥1), sont d'abord filtrés par passe-bande dans la gamme de fréquences 10-50Hz, en utilisant, par exemple, un filtre analogique de Chebyshev du quatrième ordre avec un filtre à 0,05% d'ondulation passe-bande (tel que décrit dans l'article de Weinberg, Louis; Slepian, Paul (June 1960), "Takahasi's Results on Tchebycheff and Butterworth Ladder Networks", IRE Transactions on Circuit Theory: 88-101). Le filtrage des signaux hémodynamiques dans cette étroite bande de fréquences permet d'éliminer les contributions respiratoires à basse fréquence ainsi que les contributions des ondes cardiaques acoustiques à haute fréquence. En outre, cela limite l'influence que les artefacts de mouvement pourraient avoir sur les signaux cardiaques. Ceci est particulièrement pertinent lorsque le patient implanté avec un tel dispositif bouge et, par exemple, fait de l'exercice physique.

Après le filtrage, un prétraitement supplémentaire peut être effectué sur les signaux enregistrés par le capteur hémodynamique cardiaque. Par exemple, les signaux peuvent être analysés indépendamment et classés par ordre de valeur. Un tel classement peut prendre en compte l'amplitude, la fréquence, le rapport signal/bruit, la stabilité ou la sensibilité du signal en réponse à un changement hémodynamique. Une sélection de signaux peut également être envisagée. Le meilleur signal en fonction des critères de classement peut être sélectionné pour un traitement ultérieur.

Alternativement, les signaux peuvent être combinés d'une certaine manière pour produire un signal différent, plus spécifique pour la détection de fibrillation ventriculaire. La **Figure 3** représente un accéléromètre 50 à 3 axes (x, y, z) qui pourrait être intégré dans un des dispositifs 10, 20, 30 ou être le capteur hémodynamique 40. L'axe mécanique systolique cardiaque M, représenté sur la Figure 3, est l'axe parallèle au vecteur de force maximum généré par le cœur lors de la contraction normale. Les composantes des signaux hémodynamiques d'origine selon les axes x, y, z sont projetées le long de cet axe mécanique cardiaque systolique M et additionnées pour produire un nouveau signal. Le signal hémodynamique cardiaque enregistré le long de cet axe M indique l'amplitude maximale et sensiblement le rapport signal/bruit maximum pouvant être enregistré par un capteur hémodynamique, tel qu'un accéléromètre, à la position illustrée par la Figure 3. En outre, un signal le long de cet axe mécanique cardiaque M est sensiblement un signal qui est davantage physiologique, spécifique au patient et ainsi moins arbitraire qu'un signal enregistré simplement le long de l'axe antéro-postérieur, par exemple.

Après le filtrage et le prétraitement, le signal hémodynamique sert de signal d'entrée dans le TEO. Avant de pouvoir détecter une tachyarythmie ventriculaire telle qu'une fibrillation ventriculaire selon plusieurs modes de réalisation de l'algorithme, le signal de sortie TEO est lissé avec, par exemple, une fenêtre de Hamming, comme illustré par le graphique C de la Figure 2. La fenêtre de Hamming permet de rendre les pics des signaux de sortie plus grands par rapport au bruit mais aussi de faciliter le traitement des signaux de sortie pour plusieurs modes de réalisation de l'algorithme, car elle réduit le risque de doubles détections (c'est-à-dire plusieurs pics situés à proximité les uns des autres et pouvant être à tort assimilés à un seul pic).

Après le filtrage, le prétraitement, les signaux de sortie TEO lissés servent d'entrée à des algorithmes qui prennent en compte la fréquence et/ou l'amplitude des signaux de sortie TEO dans le but de détecter la présence d'une tachyarythmie ventriculaire telle qu'une fibrillation ventriculaire. Plusieurs modes de réalisation de ces algorithmes sont décrits dans ce qui suit.

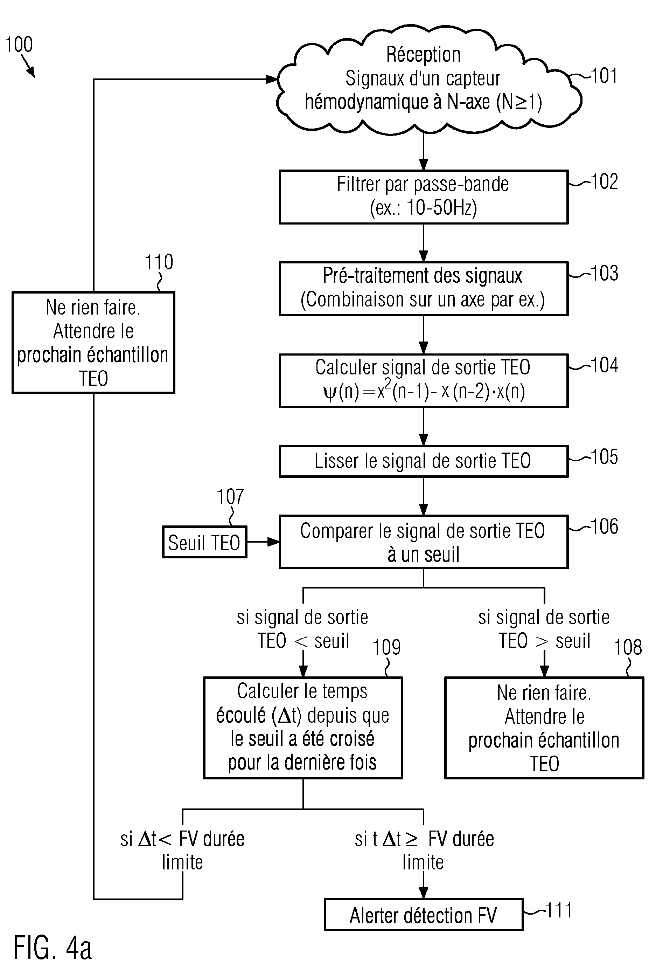

La **Figure 4a** illustre un premier mode de réalisation d'un algorithme 100.

A une étape 101, un échantillon n de signaux hémodynamiques enregistré par un capteur hémodynamique est reçu. Le capteur hémodynamique peut être un accéléromètre, un microphone, un capteur piézoélectrique, un capteur de pression ou similaire. De plus, selon une variante de l'invention, l'enregistrement de signaux hémodynamiques peut être réalisé à partir d'une combinaison de plusieurs capteurs hémodynamiques de même types (par exemple plusieurs accéléromètres) ou de catégories différentes (par exemple un capteur implanté et un capteur cutané) qui seraient positionnés à divers endroits du corps d'un patient.

A une étape 102, les signaux hémodynamiques sont filtrés par passe-bande dans la gamme de fréquences 10-50Hz afin d'éliminer les contributions respiratoires à basse fréquence et les contributions des ondes cardiaques acoustiques à haute fréquence.

A une étape 103, les signaux sont prétraités de manière à leur rendre plus pertinents pour la détection de fibrillation ventriculaire. De tels prétraitements ont été décrits en référence aux Figures 2 et 3. Par exemple, dans le cas où le capteur hémodynamique est un accéléromètre à N-axes, les composantes des signaux hémodynamiques selon les N-axes peuvent être projetés et additionnés le long d'un axe N+1, en particulier le long de l'axe mécanique cardiaque systolique M pour produire un nouveau signal dont l'amplitude est maximisée. Alternativement ou en combinaison, les signaux peuvent être filtrés par passe-bande dans la gamme de fréquences 10-50Hz, en utilisant, par exemple, un filtre analogique de Chebyshev du quatrième ordre avec un filtre à 0,05% d'ondulation passe-bande.

Les signaux hémodynamiques cardiaques filtrés et prétraités sont ensuite utilisés comme signaux d'entrée dans l'opérateur TEO. A une étape 104, les signaux hémodynamiques sont transformés par application de l'opérateur TEO qui fournit une estimation en temps réel de l'énergie nécessaire pour produire ces signaux. Cette énergie est fonction de l'amplitude et de la fréquence du signal d'origine. En particulier, l'énergie correspond au produit pondéré de l'amplitude et de la fréquence du signal d'origine.

A une étape 105, les signaux de sortie TEO sont lissés, par exemple, par application d'une fenêtre de Hamming.

A une étape 106, les signaux de sortie TEO lissés sont comparés à une valeur seuil 107. Cette valeur seuil 107 peut être statique (c'est-à-dire une seule valeur constante calculée et définie a priori) ou dynamique (c'est-à-dire qu'elle est régulièrement recalculée à l'aide de diverses valeurs du signal original ou traité). Selon le mode de réalisation dans lequel plusieurs signaux hémodynamiques cardiaques sont enregistrés par différents capteurs hémodynamiques, par exemple par un premier capteur implanté et par un deuxième capteur cutané, la valeur de seuil peut être calculée en croisant et vérifiant les signaux hémodynamiques du premier et du second capteurs hémodynamiques. Selon un autre mode de réalisation dans lequel les signaux hémodynamiques cardiaques sont enregistrés par un capteur hémodynamique à N-axes (voir l'exemple représenté par la Figure 3), la valeur de seuil peut être calculée en croisant et vérifiant les signaux hémodynamiques respectivement enregistrés selon chaque axe.

Si le signal de sortie TEO est supérieur à la valeur seuil 107, aucune action n'est entreprise à l'étape 108 et le prochain échantillon de signaux de sortie TEO lissé est prêt à être pris en compte.

Si, toutefois, le signal de sortie TEO est inférieur à la valeur seuil 107, le temps écoulé « Δt » entre l'échantillon actuel et le dernier échantillon qui a été supérieur au seuil 107 est calculé à une étape 109.

Si le temps écoulé « Δt » est inférieur à une limite de temps spécifique caractéristique d'une fibrillation ventriculaire (« FV durée limite » à la Figure 4a), à l'étape 110 aucune action n'est entreprise et la réception du prochain échantillon n+1 de signaux de sortie TEO est attendue.

Si toutefois, le temps écoulé « Δt » est supérieur ou égal à la « FV durée limite », une fibrillation ventriculaire est détectée et une alerte est déclenchée à l'étape 111. Comme pour le seuil, la valeur spécifique de la « FV durée limite » peut également être statique (c'est-à-dire une valeur unique inchangée et définie a priori) ou dynamique (c'est-à-dire qu'elle est régulièrement recalculée en fonction du signal d'origine ou du signal de sortie).

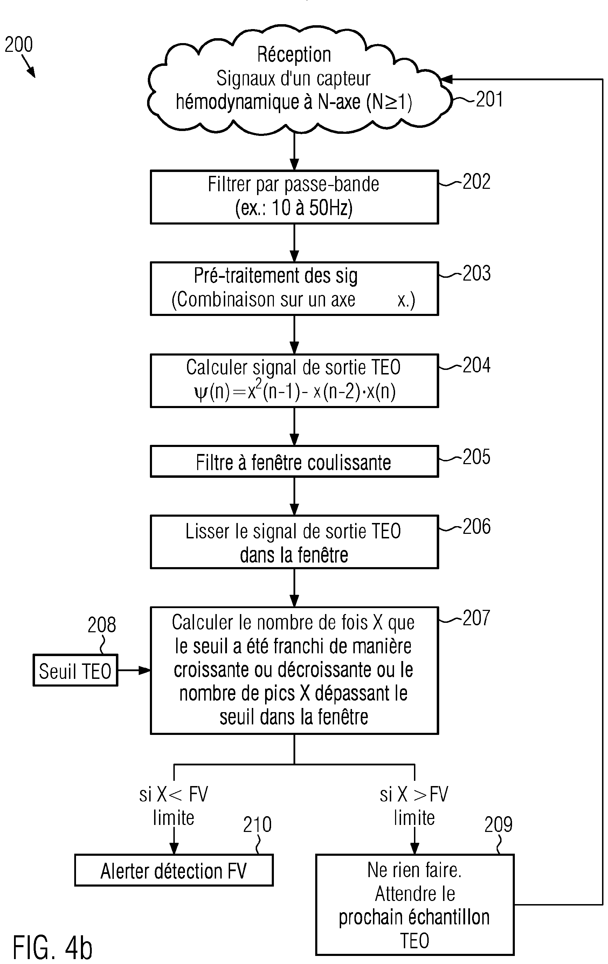

La **Figure 4b** illustre un deuxième mode de réalisation d'un algorithme 200.

De manière similaire à l'algorithme 100, un échantillon n de signaux hémodynamiques enregistré par un capteur hémodynamique est reçu à l'étape 201. Le capteur hémodynamique peut être un accéléromètre, un microphone, un capteur piézoélectrique, un capteur de pression ou similaire. De plus, selon une variante de l'invention, l'enregistrement de signaux hémodynamiques peut être réalisé à partir d'une combinaison de plusieurs capteurs hémodynamiques de même types (par exemple plusieurs accéléromètres) ou de catégories différentes (par exemple un capteur implanté et un capteur cutané) qui seraient positionnés à divers endroits du corps d'un patient.

Puis, à une étape 202, les signaux hémodynamiques sont filtrés par passe-bande dans la gamme de fréquences 10-50Hz afin d'éliminer les contributions respiratoires à basse fréquence et les contributions des ondes cardiaques acoustiques à haute fréquence. A une étape 203, les signaux sont prétraités de manière à les rendre plus pertinents pour la détection de fibrillation ventriculaire. De tels prétraitements ont été décrits en référence aux Figures 2, 3 et 4a. A une étape 204, les signaux hémodynamiques sont transformés par application de l'opérateur TEO.

Selon le deuxième mode de réalisation, à une étape 205, les signaux de sortie TEO sont filtrés par application d'un filtre à fenêtre glissante (appelée « *sliding window* » en anglais) dont la longueur de la « fenêtre » peut être statique (c'est-à-dire une seule valeur inchangée calculée et définie a priori) ou dynamique (c'est-à-dire qu'elle est régulièrement recalculée à l'aide du signal original ou de sortie).

La « fenêtre » est glissée (c'est-à-dire déplacée vers l'échantillon suivant ou précédant sans « sauter » plus d'un échantillon à la fois, comme c'est le cas pour les fenêtres dites « jumping window » ou « saut de fenêtre »), sur le dernier échantillon de signaux de sortie TEO qui sont lissés à une étape 206 à l'aide, par exemple, d'une fenêtre de Hamming.

A une étape 207, les signaux de sortie TEO lissés sont comparés à une valeur seuil 208 à l'intérieur de la « fenêtre ». Cette valeur seuil 208 peut être statique (c'est-à-dire une seule valeur inchangée calculée et définie a priori) ou dynamique (c'est-à-dire qu'elle est régulièrement recalculée à l'aide du signal de sortie TEO lissé). A l'étape 207, le nombre de fois X que le seuil 208 a été franchi de manière croissante ou décroissante ou le nombre de pics X dépassant le seuil 208 à l'intérieur de la « fenêtre » est calculé.

Si le nombre X est supérieur à un nombre limite spécifique (« FV Limite » dans la Figure 4b), à l'étape 209 aucune action n'est entreprise et la réception du prochain échantillon n+1 de signaux est attendue.

Si le nombre X est inférieur au nombre limite spécifique « FV limite », une fibrillation ventriculaire est détectée et une alerte est déclenchée à l'étape 210. Comme pour le seuil 208, la valeur spécifique du nombre « FV limite » peut également être statique (c'est-à-dire une valeur unique inchangée et définie a priori) ou dynamique (c'est-à-dire qu'elle est régulièrement recalculée en fonction du signal d'origine ou du signal de sortie). Selon le mode de réalisation dans lequel plusieurs signaux hémodynamiques cardiaques sont enregistrés par différents capteurs hémodynamiques, par exemple par un premier capteur implanté et par un deuxième capteur cutané, la valeur de seuil peut être calculée en croisant et vérifiant les signaux hémodynamiques du premier et du second capteurs hémodynamiques. Selon un autre mode de réalisation dans lequel les signaux hémodynamiques cardiaques sont enregistrés par un capteur hémodynamique à N-axes (voir l'exemple représenté par la Figure 3), la valeur de seuil peut être calculée en croisant et vérifiant les signaux hémodynamiques respectivement enregistrés selon chaque axe

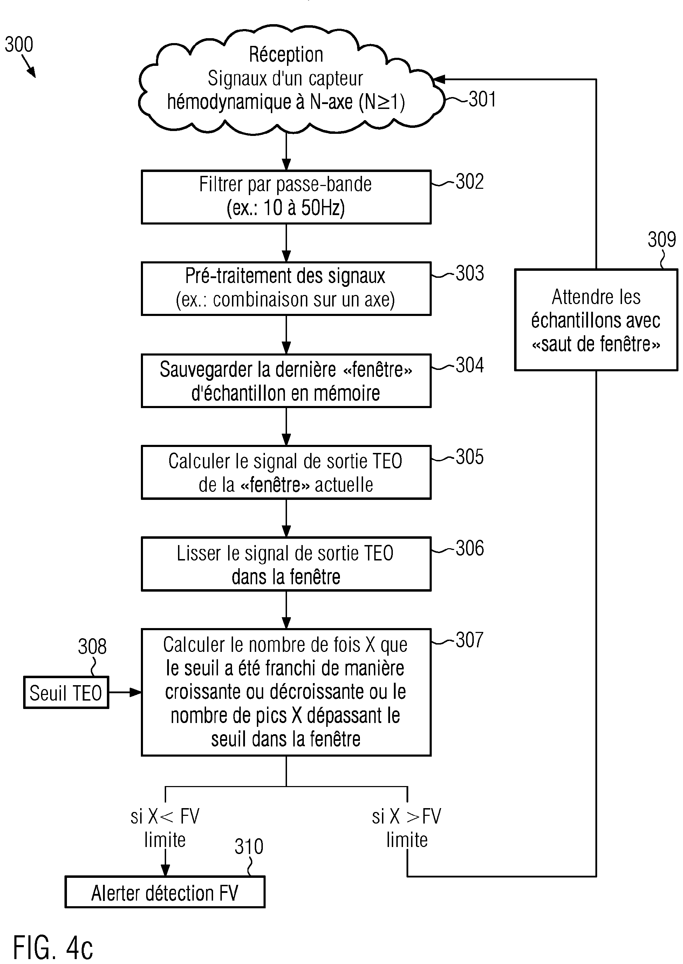

La **Figure 4c** illustre un troisième mode de réalisation d'un algorithme 300.

De manière similaire à l'algorithme 200, un échantillon n de signaux hémodynamiques enregistré par un capteur hémodynamique est reçu à une étape 301. Le capteur hémodynamique peut être un accéléromètre, un microphone, un capteur piézoélectrique, un capteur de pression ou similaire. De plus, selon une variante de l'invention, l'enregistrement de signaux hémodynamiques peut être réalisé à partir d'une combinaison de plusieurs capteurs hémodynamiques de même types (par exemple plusieurs accéléromètres) ou de catégories différentes (par exemple un capteur implanté et un capteur cutané) qui seraient positionnés à divers endroits du corps d'un patient.

Puis, à une étape 302, les signaux hémodynamiques sont filtrés par passe-bande dans la gamme de fréquences 10-50Hz afin d'éliminer les contributions respiratoires à basse fréquence et les contributions des ondes cardiaques acoustiques à haute fréquence. A une étape 303, les signaux sont prétraités de manière à les rendre plus pertinents pour la détection de fibrillation ventriculaire. De tels prétraitements ont été décrits en référence aux Figures 2, 3 et 4a.

Selon le troisième mode de réalisation, à une étape 304, après le filtrage passe-bande et le prétraitement, les derniers échantillons du signal hémodynamique cardiaque de la « fenêtre » sont stockés dans la mémoire. Cette fenêtre est du type « saut de fenêtre » (« jumping window » en anglais) et la longueur de la « fenêtre » peut être statique (c'est-à-dire une seule valeur inchangée calculée et définie a priori) ou dynamique (c'est-à-dire qu'elle est régulièrement recalculée à l'aide du signal original ou de sortie).

A une étape 305, les signaux hémodynamiques sont transformés par application de l'opérateur TEO. A l'étape 305, les signaux de sortie TEO sont calculés dans la « fenêtre » actuelle.

A une étape 306, les signaux de sortie TEO sont lissés dans la « fenêtre » actuelle à l'aide, par exemple, d'une fenêtre de Hamming.

A une étape 307, les signaux de sortie TEO lissés sont comparés à une valeur seuil 308 à l'intérieur de la « fenêtre ». Cette valeur seuil 308 peut être statique (c'est-à-dire une seule valeur inchangée calculée et définie a priori) ou dynamique (c'est-à-dire qu'elle est régulièrement recalculée à l'aide du signal de sortie TEO lissé). Selon le mode de réalisation dans lequel plusieurs signaux hémodynamiques cardiaques sont enregistrés par différents capteurs hémodynamiques, par exemple par un premier capteur implanté et par un deuxième capteur cutané, la valeur de seuil peut être calculée en croisant et vérifiant les signaux hémodynamiques du premier et du second capteurs hémodynamiques. Selon un autre mode de réalisation dans lequel les signaux hémodynamiques cardiaques sont enregistrés par un capteur hémodynamique à N-axes (voir l'exemple représenté par la Figure 3), la valeur de seuil peut être calculée en croisant et vérifiant les signaux hémodynamiques respectivement enregistrés selon chaque axe. A l'étape 307, le nombre de fois X que le seuil 308 a été franchi de manière croissante ou décroissante ou le nombre de pics X dépassant le seuil 308 à l'intérieur de la « fenêtre » est calculé.

Si le nombre X est supérieur à un nombre limite spécifique (« FV limite » dans la Figure 4c) aucune fibrillation ventriculaire n'a été détectée : à l'étape 309 l'algorithme 300 attend un nombre spécifique « de sauts d'échantillons » avant d'envisager le traitement de la fenêtre suivante. Entre-temps, le signal prétraité est toujours en cours d'enregistrement et sauvegardé en mémoire à l'étape 304 pour pouvoir être pris en compte dans la fenêtre suivante.

Si le nombre X est inférieur au nombre limite spécifique « FV limite », une fibrillation ventriculaire est détectée et une alerte est déclenchée à l'étape 310. Comme pour le seuil 308, la valeur spécifique du nombre « FV limite » peut également être statique (c'est-à-dire une valeur unique inchangée et définie a priori) ou dynamique (c'est-à-dire qu'elle est régulièrement recalculée en fonction du signal d'origine ou du signal de sortie).

L'algorithme 300 permet un coût de calcul inférieur aux algorithmes 100 et 200, car il calcule uniquement le signal de sortie TEO à l'intérieur de la fenêtre pour tous les « sauts échantillons » plutôt que de manière continue.

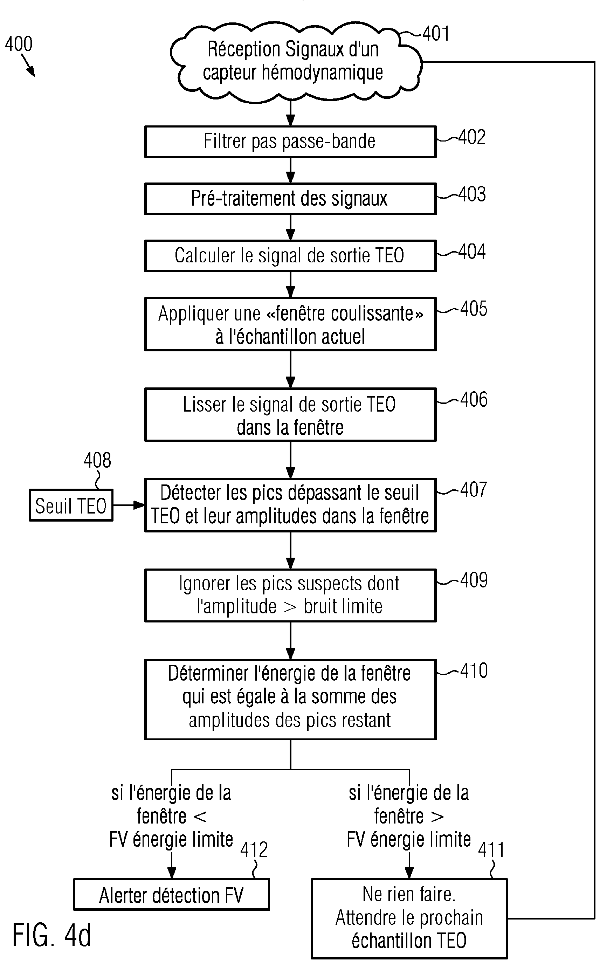

La **Figure 4d** illustre un quatrième mode de réalisation d'un algorithme 400.

A la différence des algorithmes 100, 200, 300 précédents, l'algorithme 400 prend en compte le nombre de pics dépassant le seuil dans une fenêtre dite « glissante » (pour « sliding window » en anglais ou être un «saut de fenêtre » (pour «jumping window» en anglais), ainsi que leurs amplitudes.

A une étape 401, un échantillon n de signaux hémodynamiques enregistré par un capteur hémodynamique est reçu. Le capteur hémodynamique peut être un accéléromètre, un microphone, un capteur piézoélectrique, un capteur de pression ou similaire. De plus, selon une variante de l'invention, l'enregistrement de signaux hémodynamiques peut être réalisé à partir d'une combinaison de plusieurs capteurs hémodynamiques de même types (par exemple plusieurs accéléromètres) ou de catégories différentes (par exemple un capteur implanté et un capteur cutané) qui seraient positionnés à divers endroits du corps d'un patient.

Puis, à une étape 402, les signaux hémodynamiques sont filtrés par passe-bande dans la gamme de fréquences 10-50Hz afin d'éliminer les contributions respiratoires à basse fréquence et les contributions des ondes cardiaques acoustiques à haute fréquence.

A une étape 403, les signaux sont prétraités de manière à les rendre plus pertinents pour la détection de fibrillation ventriculaire. De tels prétraitements ont été décrits en référence aux Figures 2, 3 et 4a.

A une étape 404, les signaux hémodynamiques sont transformés par application de l'opérateur TEO.

Selon le quatrième mode de réalisation, les signaux de sortie TEO sont considérés dans une fenêtre « glissante » à l'étape 405. La valeur de la longueur de la fenêtre peut être statique (c'est-à-dire une valeur unique constante calculée et définie a priori) ou dynamique (c'est-à-dire qu'elle est régulièrement recalculée à l'aide du signal original ou traité).

A l'étape 406, les signaux de sortie TEO sont lissés dans ladite fenêtre à l'aide, par exemple, d'une fenêtre de Hamming.

A l'étape 407, les pics des signaux de sortie TEO lissés dépassant une valeur seuil TEO 408 dans la fenêtre et leurs amplitudes sont déterminés. Cette valeur seuil 408 peut être statique (c'est-à-dire une seule valeur inchangée calculée et définie a priori) ou dynamique (c'est-à-dire qu'elle est régulièrement recalculée à l'aide du signal de sortie TEO lissé). Selon le mode de réalisation dans lequel plusieurs signaux hémodynamiques cardiaques sont enregistrés par différents capteurs hémodynamiques, par exemple par un premier capteur implanté et par un deuxième capteur cutané, la valeur de seuil peut être calculée en croisant et vérifiant les signaux hémodynamiques du premier et du second capteurs hémodynamiques. Selon un autre mode de réalisation dans lequel les signaux hémodynamiques cardiaques sont enregistrés par un capteur hémodynamique à N-axes (voir l'exemple représenté par la Figure 3), la valeur de seuil peut être calculée en croisant et vérifiant les signaux hémodynamiques respectivement enregistrés selon chaque axe.

Les pics comportant des amplitudes particulièrement grandes sont suspectés d'être des bruits externes et parasites et pourraient alors induire en erreur le traitement des signaux. Par conséquent, à l'étape 409, tous les pics dont l'amplitude dépasse une valeur spécifique (« bruit limite » dans Figure 4d), sont ignorés. La valeur de « bruit limite » peut être statique (c'est-à-dire une seule valeur inchangée calculée et définie a priori) ou dynamique (c'est-à-dire qu'elle est régulièrement recalculée à l'aide du signal de sortie TEO lissé).

A l'étape 410, les amplitudes des pics restants sont additionnées pour ne donner qu'une seule valeur appelée « énergie de la fenêtre ».

Si la valeur de « l'énergie de fenêtre » est supérieure à une valeur spécifique dite « FV énergie limite », à l'étape 411 aucune action n'est entreprise et la réception du prochain échantillon n+1 de signaux est attendue.

Si la valeur de « l'énergie de fenêtre » est inférieure à la valeur spécifique dite « FV énergie limite », une fibrillation ventriculaire est détectée et une alerte est déclenchée à l'étape 412. Comme pour le seuil 408, la valeur spécifique du nombre « FV énergie limite » peut également être statique (c'est-à-dire une valeur unique inchangée et définie a priori) ou dynamique (c'est-à-dire qu'elle est régulièrement recalculée en fonction du signal d'origine ou du signal de sortie). Ce seuil « FV énergie limite » peut être interprété comme l'énergie minimale requise pour garantir une perfusion systémique (c'est-à-dire la circulation du sang dans le cerveau et le corps) suffisante.

Les différents modes de réalisation du dispositif et du procédé 100, 200, 300, 400 selon la présente invention peuvent en outre être configurés pour déclencher une opération de défibrillation à la suite de la détection d'une fibrillation ventriculaire, suite aux étapes «Alerter détection FV » 111, 210, 310, 412, notamment au moyen d'un défibrillateur manuel ou automatique. De plus, le procédé et le dispositif de la présente invention peuvent être configurés pour vérifier que l'opération de défibrillation a été efficace, c'est-à-dire vérifier si l'activité mécanique du cœur a été rétablie ou non après l'opération de défibrillation. Pour se faire, après un laps de temps prédéterminé permettant le rétablissement du rythme cardiaque normal suite à l'opération de défibrillation, un signal cardiaque hémodynamique est détecté au moyen d'un capteur hémodynamique et est traité notamment par l'application de l'opérateur TEO, tel que décrit par rapport aux Figures 4a à 4d. Un signal cardiaque hémodynamique de sortie « post-traitement » est ainsi défini. De la même maniéré que celle décrite par rapport à au moins une des Figures 4a à 4d, ledit signal cardiaque hémodynamique de sortie « post-traitement » est comparé à un seuil prédéterminé afin de déterminer si l'opération de défibrillation a permis de traiter efficacement la fibrillation ventriculaire. Dans le cas négatif, une nouvelle opération de défibrillation peut être déclenchée au cours de laquelle un signal de défibrillation électrique thérapeutique (choc électrique) est délivré au patient.

Les modes de réalisation décrits sont simplement des configurations possibles et il faut garder à l'esprit que les caractéristiques individuelles des différents modes de réalisation peuvent être combinées entre elles ou fournies indépendamment les unes des autres.

De plus, chacun des algorithmes 100, 200, 300, 400 peut comprendre des étapes supplémentaires avant, entre ou après les étapes qui ont décrites en référence aux Figures 4a, 4b, 4c et 4d.

## Revendications

1. Un dispositif médical, en particulier un dispositif médical implantable, comprenant :
au moins un capteur hémodynamique implantable ou non-implantable configuré pour détecter des signaux cardiaques hémodynamiques;
un contrôleur configuré pour traiter et analyser les signaux hémodynamiques cardiaques détectés et/ou des signaux dérivés de signaux hémodynamiques cardiaques détectés en appliquant auxdits signaux un Opérateur d'Energie Teager (TEO) défini par:
TEO {x (n)} =Ψ (n) = x².(n-1)-x(n-2).x(n) où « x (n) » est un signal hémodynamique cardiaque, « Ψ (n) » est le signal hémodynamique de sortie et « n » fait référence à un échantillon prédéterminé ;
ledit contrôleur comprenant en outre au moins un algorithme configuré pour déterminer la nécessité d'une opération de défibrillation en prenant en compte le au moins un signal hémodynamique de sortie.

2. Le dispositif médical selon la revendication 1, dans lequel le au moins un capteur hémodynamique est un accéléromètre implantable ou non-implantable, un microphone, un capteur piézoélectrique ou un capteur de pression apte à détecter des signaux cardiaques hémodynamiques, en particulier des sons cardiaques.

3. Le dispositif médical selon la revendication 1 ou 2, dans lequel le au moins un algorithme du contrôleur est configuré pour déterminer la nécessité d'une opération de défibrillation si au moins une caractéristique des signaux hémodynamiques de sortie ne franchit pas un seuil prédéterminé pendant un lapse de temps qui est supérieur à une durée associée à un épisode de fibrillation ventriculaire.

4. Le dispositif médical selon la revendication 3, dans lequel la au moins une caractéristique du signal hémodynamique de sortie est l'amplitude, l'énergie, la valeur moyenne ou la valeur médiane, ou la valeur quadratique moyenne dudit signal.

5. Le dispositif médical selon l'une des revendications 3 ou 4, dans lequel l'amplitude des signaux hémodynamiques de sortie, et/ou
le nombre de fois que chacun des signaux hémodynamiques de sortie franchit de manière ascendante ou descendante le seuil prédéterminé, et/ou le nombre de pics de chaque signal qui dépasse le seuil prédéterminé pendant le lapse de temps prédéfini,
est/sont pris en compte pour déterminer la présence ou non d'une fibrillation ventriculaire.

6. Le dispositif médical selon l'une des revendications précédentes, comprenant en outre un défibrillateur, en particulier un défibrillateur implantable, configuré pour générer un signal de défibrillation électrique ; et
au moins une électrode connectée audit défibrillateur par une sonde configurée pour délivrer ledit signal de défibrillation électrique à un patient
lorsque la nécessité de déclencher une opération défibrillation est déterminée au moyen du contrôleur du dispositif médical.

7. Le dispositif médical selon la revendication 6, dans lequel le contrôleur est configuré pour déterminer la nécessité de déclencher une opération de défibrillation en comparant les signaux hémodynamiques cardiaques de sortie à au moins un signal électrophysiologique détecté de manière indépendante aux signaux hémodynamiques cardiaques au moyen de la au moins une électrode.

8. Le dispositif médical selon l'une des revendications 1 à 6, dans lequel le contrôleur est configuré pour déterminer la nécessité d'une opération de défibrillation en prenant en compte uniquement des signaux hémodynamiques cardiaques.

9. Le dispositif médical selon l'une des revendications précédentes, dans lequel le contrôleur est configuré pour filtrer par passe-bande les signaux cardiaques hémodynamiques détectés et/ou des signaux dérivés des signaux hémodynamiques cardiaques détectés dans une gamme de 1 à 100 Hz, en particulier de 7,5 à 49 Hz.

10. Le dispositif médical selon l'une des revendications précédentes, dans lequel le contrôleur est en outre configuré pour filtrer les signaux de sortie par application d'une fonction de fenêtrage, en particulier une fenêtre de Hamming.

11. Le dispositif médical selon l'une des revendications 3 à 10, dans lequel le contrôleur est configuré pour déterminer la nécessité d'une opération de défibrillation et/ou déclencher une opération de défibrillation selon :
un seuil statique prédéfini, ou
selon un seuil dynamique, recalculé périodiquement et dérivé d'une ou de plusieurs caractéristiques des signaux hémodynamiques cardiaques ou de leur représentation par l'Opérateur d'Energie Teager.

12. Procédé pour traiter de signaux cardiaques hémodynamiques détectés par au moins un capteur hémodynamique d'un dispositif médical, en particulier d'un dispositif médical implantable, comprenant :
au moins une étape de traitement des signaux hémodynamiques détectés et/ou de signaux dérivés des signaux hémodynamiques cardiaques détectés qui comprend l'application d'un Opérateur d'Energie Teager (TEO) défini par: TEO {x (n)} =Ψ (n) = x².(n-1)-x(n-2).x(n) où « x (n) » est un signal hémodynamique cardiaque détecté, « Ψ (n) » est le signal cardiaque hémodynamique de sortie et « n » fait référence à un échantillon prédéterminé ;
et comprenant en outre une étape au cours de laquelle la présence ou non d'une fibrillation ventriculaire est déterminée en prenant en compte le ou les signaux hémodynamiques cardiaques de sortie par rapport à un seuil prédéterminé.

13. Le procédé pour traiter de signaux cardiaques hémodynamiques selon la revendication 12, dont l'étape de traitement des signaux est précédée d'une étape de prétraitement des signaux à laquelle les signaux hémodynamiques détectés et/ou les signaux dérivés des signaux hémodynamiques cardiaques détectés sont filtrés par passe-bande, en particulier dans une gamme de 1 à 100 Hz, plus en particulier de 7,5 à 49 Hz.

14. Le procédé pour traiter de signaux cardiaques hémodynamiques selon la revendication 12 ou 13, dont l'étape de traitement des signaux est suivie d'une étape de post-traitement des signaux à laquelle les signaux hémodynamiques de sortie sont filtrés par application d'une fonction de fenêtrage, en particulier par application d'une fenêtre de Hamming.

15. Le procédé pour traiter de signaux cardiaques hémodynamiques selon la revendication 12, 13 ou 14, dont le moins un capteur hémodynamique est un accéléromètre à N-axes (N >= 1) implantable ou non-implantable, un microphone, un capteur piézoélectrique ou un capteur de pression apte à détecter des signaux cardiaques hémodynamiques, en particulier des sons cardiaques.

16. Le procédé pour traiter de signaux cardiaques hémodynamiques selon la revendication 15, dont les N signaux cardiaques détectés par l'accéléromètre à N-axes sont combinés dans un nouveau signal selon un axe N+1, l'axe N+1 étant déterminé de sorte que l'amplitude et/ou le rapport signal sur bruit et/ou la stabilité et/ou un paramètre physiologique pertinent du nouveau signal est maximale selon l'axe N+1.

17. Le procédé pour traiter de signaux cardiaques hémodynamiques selon l'une des revendications 12 à 16, dont le seuil prédéterminé est un seuil statique prédéfini, ou
un seuil dynamique, recalculé périodiquement et dérivé d'une ou de plusieurs caractéristiques des signaux hémodynamiques cardiaques ou de leur représentation par l'Opérateur d'Energie Teager.

18. Le procédé pour traiter de signaux cardiaques hémodynamiques selon l'une des revendications 12 à 17, dans lequel l'amplitude, et/ou
le nombre de fois que chacun des signaux hémodynamiques de sortie franchit de manière ascendante ou descendante le seuil prédéterminé, et/ou le nombre de pics de chaque signal qui dépasse le seuil prédéterminé pendant un lapse de temps prédéfini,
est/sont pris en compte pour déterminer la présence ou non d'une fibrillation ventriculaire.

19. Le procédé pour traiter de signaux cardiaques hémodynamiques selon l'une des revendications 12 à 18, dans lequel la présence ou non d'une fibrillation ventriculaire est déterminée en prenant en compte uniquement des signaux hémodynamiques cardiaques.

20. Le procédé pour traiter de signaux cardiaques hémodynamiques selon l'une des revendications 12 à 18, dans lequel la présence ou non d'une fibrillation ventriculaire est établie en comparant le signal hémodynamique de sortie à au moins un signal électrophysiologique détecté au moyen d'une électrode du dispositif médical.

21. Logiciel de traitement de signaux cardiaques hémodynamiques détectés par au moins un capteur hémodynamique d'un dispositif médical, en particulier d'un dispositif médical implantable, **caractérisé en ce qu'**il comprend des instructions aptes, lorsqu'elles sont exécutées par le dispositif médical, à exécuter le traitement des signaux hémodynamiques détectés et/ou des signaux dérivés des signaux hémodynamiques cardiaques détectés par application d'un Opérateur d'Energie Teager (TEO) défini par:
TEO {x (n)} =Ψ (n) = x² (n-1) -x (n-2) x (n) où «x (n)» est le signal hémodynamique cardiaque détecté, «Ψ (n)» est le signal de sortie et «n» fait référence à un échantillon prédéterminé ; et à comparer au moins une caractéristique du signal de sortie à un seuil prédéterminé ; et à déclencher une alerte de fibrillation ventriculaire lorsque la au moins une caractéristique du
signal de sortie ne franchit pas le seuil prédéterminé pendant un lapse de temps prédéfini.

22. Le logiciel de traitement selon la revendication 21, qui comprend en outre le prétraitement des signaux hémodynamiques cardiaques détectés et/ou des signaux dérivés des signaux hémodynamiques cardiaques détectés par filtration passe-bande, en particulier dans une gamme de 1 à 100 Hz, plus en particulier de 7,5 à 49 Hz.

23. Le logiciel de traitement selon la revendication 21 ou 22, qui comprend en outre le post-traitement du signal de sortie par application d'une fonction de fenêtrage, en particulier une fenêtre de Hamming.

## Patentansprüche

1. Medizinisches Produkt, insbesondere ein implantierbares medizinisches Produkt, das Folgendes umfasst: zumindest einen implantierbaren oder nicht implantierbaren hämodynamischen Sensor, der konfiguriert ist, um hämodynamische Herzsignale zu erfassen;
eine Steuerung, die konfiguriert ist, um die erfassten hämodynamischen Herzsignale und/oder von erfassten hämodynamischen Herzsignalen abgeleitete Signale zu verarbeiten und zu analysieren, indem auf besagte Signale ein Teager Energy Operator (TEO) angewendet wird, der definiert ist durch:
TEO {x (n)} = Ψ (n) = x² (n-1) -x (n-2) x (n), wobei "x (n)" ein hämodynamisches Herzsignal ist, "ψ (n)" das hämodynamische Ausgangssignal ist und "n" sich auf eine vorbestimmte Probe bezieht;
wobei besagte Steuerung ferner zumindest einen Algorithmus umfasst, der konfiguriert ist, um unter Berücksichtigung des zumindest einen hämodynamischen Ausgangssignals die Notwendigkeit eines Defibrillationsvorgangs zu bestimmen.

2. Medizinisches Produkt nach Anspruch 1, worin der zumindest eine hämodynamische Sensor ein implantierbarer oder nicht implantierbarer Beschleunigungsmesser, ein Mikrofon, ein piezoelektrischer Sensor oder ein Drucksensor ist, der in der Lage ist, hämodynamische Herzsignale, insbesondere Herzgeräusche, zu erfassen.

3. Medizinisches Produkt nach Anspruch 1 oder 2, worin der zumindest eine Algorithmus der Steuerung konfiguriert ist, um die Notwendigkeit eines Defibrillationsvorgangs zu bestimmen, wenn zumindest eine Eigenschaft der hämodynamischen Ausgangssignale einen vorbestimmten Schwellenwert während eines Zeitraums nicht überschreitet, der größer ist als eine Dauer, die mit einer Episode von Kammerflimmern verbunden ist.

4. Medizinisches Produkt nach Anspruch 3, worin die zumindest eine Eigenschaft des hämodynamischen Ausgangssignals die Amplitude, die Energie, der Mittelwert oder der Medianwert oder der quadratische Mittelwert des besagten Signals ist.

5. Medizinisches Produkt nach einem der Ansprüche 3 oder 4, worin die Amplitude der hämodynamischen Ausgangssignale und/oder
die Anzahl, wie häufig jedes der hämodynamischen Ausgangssignale den vorbestimmten Schwellenwert über- oder unterschreitet, und/oder die Anzahl der Peaks jedes Signals, das den vorgegebenen Schwellenwert während des vorgegebenen Zeitraums überschreitet,
bei der Bestimmung des Vorhandenseins oder Nichtvorhandenseins von Kammerflimmern berücksichtigt wird/werden.

6. Medizinisches Produkt nach einem der vorstehenden Ansprüche, das ferner einen Defibrillator, insbesondere einen implantierbaren Defibrillator, der konfiguriert ist, um ein elektrisches Defibrillationssignal zu erzeugen; und
zumindest eine Elektrode, die mit besagtem Defibrillator durch eine Sonde verbunden ist, die konfiguriert ist, um besagtes elektrisches Defibrillationssignal an einen Patienten zu liefern, wenn die Notwendigkeit, einen Defibrillationsvorgang einzuleiten, mittels der Steuerung des medizinischen Produkts bestimmt wird, umfasst.

7. Medizinisches Produkt nach Anspruch 6, worin die Steuerung konfiguriert ist, um die Notwendigkeit, einen Defibrillationsvorgang einzuleiten, durch Vergleich der hämodynamischen Herzausgangssignale mit zumindest einem, mittels der zumindest einen Elektrode unabhängig von den hämodynamischen Herzsignalen erfassten, elektrophysiologischen Signal zu bestimmen.

8. Medizinisches Produkt nach einem der Ansprüche 1 bis 6, worin die Steuerung konfiguriert ist, um die Notwendigkeit eines Defibrillationsvorgangs unter Berücksichtigung ausschließlich hämodynamischer Herzsignale zu bestimmen.

9. Medizinisches Produkt nach einem der vorstehenden Ansprüche, worin die Steuerung konfiguriert ist, um die erfassten hämodynamischen Herzsignale und/oder von den erfassten hämodynamischen Herzsignalen abgeleitete Signale mittels Bandpass in einem Bereich von 1 bis 100 Hz, insbesondere von 7,5 bis 49 Hz, zu filtern.

10. Medizinisches Produkt nach einem der vorstehenden Ansprüche, worin die Steuerung ferner konfiguriert ist, um die Ausgangssignale durch Anwenden einer Fensterfunktion, insbesondere eines Hamming-Fensters, zu filtern.

11. Medizinisches Produkt nach einem der Ansprüche 3 bis 10, worin die Steuerung konfiguriert ist, um die Notwendigkeit eines Defibrillationsvorgangs zu bestimmen und/oder einen Defibrillationsvorgang einzuleiten gemäß:
einem vordefinierten statischen Schwellenwert, oder
gemäß einem dynamischen Schwellenwert, der periodisch neu berechnet und aus einer oder mehreren Eigenschaften der hämodynamischen Herzsignale oder ihrer Darstellung durch den Teager Energy Operator abgeleitet wird.

12. Verfahren zum Verarbeiten hämodynamischer Herzsignale, die von zumindest einem hämodynamischen Sensor eines medizinischen Produktes, insbesondere eines implantierbaren medizinischen Produktes, erfasst werden, das Folgendes umfasst:
zumindest einen Schritt des Verarbeitens der erfassten hämodynamischen Signale und/oder von von den erfassten hämodynamischen Herzsignalen abgeleiteten Signalen, der das Anwenden eines Teager Energy Operator (TEO) umfasst, der definiert ist durch:
TEO {x (n)} = ψ (n) = x² (n-1) -x (n-2) x (n), wobei "x (n)" ein erfasstes hämodynamisches Herzsignal ist, "ψ (n)" das hämodynamische Herzausgangssignal ist und "n" sich auf eine vorbestimmte Probe bezieht;
und ferner einen Schritt umfasst, bei dem unter Berücksichtigung des oder der hämodynamischen Herzausgangssignale in Bezug auf einen vorbestimmten Schwellenwert das Vorhandensein oder Nichtvorhandensein von Kammerflimmern bestimmt wird.

13. Verfahren zur Verarbeitung hämodynamischer Herzsignale nach Anspruch 12, wobei dem Signalverarbeitungsschritt ein Signalvorverarbeitungsschritt vorangeht, bei dem die erfassten hämodynamischen Signale und/oder die aus den erfassten hämodynamischen Herzsignalen abgeleiteten Signale mittels Bandpass insbesondere in einem Bereich von 1 bis 100 Hz, ganz insbesondere von 7,5 bis 49 Hz, gefiltert werden.

14. Verfahren zum Verarbeiten hämodynamischer Herzsignale nach Anspruch 12 oder 13, wobei dem Signalverarbeitungsschritt ein Signalnachverarbeitungsschritt folgt, bei dem die hämodynamischen Ausgangssignale durch Anwenden einer Fensterfunktion, insbesondere durch Anwenden eines Hamming-Fensters, gefiltert werden.

15. Verfahren zum Verarbeiten hämodynamischer Herzsignale nach Anspruch 12, 13 oder 14, wobei der zumindest eine hämodynamische Sensor ein implantierbarer oder nicht implantierbarer N-Achsen-Beschleunigungsmesser (N >= 1), ein Mikrofon, ein piezoelektrischer Sensor oder ein Drucksensor ist, der in der Lage ist, hämodynamische Herzsignale, insbesondere Herzgeräusche, zu erfassen.

16. Verfahren zum Verarbeiten hämodynamischer Herzsignale nach Anspruch 15, wobei die von dem N-Achsen-Beschleunigungsmesser erfassten N Herzsignale in einem neuen Signal gemäß einer N+1-Achse kombiniert werden, wobei die N+1-Achse derart bestimmt wird, dass die Amplitude und/oder das Signal-Rausch-Verhältnis und/oder die Stabilität und/oder ein relevanter physiologischer Parameter des neuen Signals gemäß der N+1-Achse maximal ist.

17. Verfahren zum Verarbeiten hämodynamischer Herzsignale nach einem der Ansprüche 12 bis 16, wobei der vorbestimmte Schwellenwert ein vordefinierter statischer Schwellenwert oder ein periodisch neu berechneter und von einer oder mehreren Eigenschaften der hämodynamischen Herzsignale oder deren Darstellung durch den Teager Energy Operator abgeleiteter dynamischer Schwellenwert ist.

18. Verfahren zum Verarbeiten hämodynamischer Herzsignale nach einem der Ansprüche 12 bis 17, worin die Amplitude und/oder
die Anzahl, wie häufig jedes der hämodynamischen Ausgangssignale den vorbestimmten Schwellenwert über- oder unterschreitet, und/oder die Anzahl der Peaks jedes Signals, das den vorbestimmten Schwellenwert während eines vorbestimmten Zeitraums überschreitet,
bei der Bestimmung des Vorhandenseins oder Nichtvorhandenseins von Kammerflimmern berücksichtigt wird/werden.

19. Verfahren zum Verarbeiten hämodynamischer Herzsignale nach einem der Ansprüche 12 bis 18, worin das Vorhandensein oder Nichtvorhandensein von Kammerflimmern unter Berücksichtigung ausschließlich hämodynamischer Herzsignale bestimmt wird.

20. Verfahren zum Verarbeiten hämodynamischer Herzsignale nach einem der Ansprüche 12 bis 18, worin das Vorhandensein oder Nichtvorhandensein von Kammerflimmern durch Vergleichen des hämodynamischen Ausgangssignals mit zumindest einem mittels einer Elektrode des medizinischen Produkts erfassten elektrophysiologischen Signal festgestellt wird.

21. Software zum Verarbeiten hämodynamischer Herzsignale, die von zumindest einem hämodynamischen Sensor eines medizinischen Produktes, insbesondere eines implantierbaren medizinischen Produktes, erfasst werden, **dadurch gekennzeichnet, dass** sie Anweisungen enthält, die, wenn sie von dem medizinischen Produkt ausgeführt werden, in der Lage sind, die Verarbeitung der erfassten hämodynamischen Signale und/oder der von den erfassten hämodynamischen Signalen abgeleiteten Signale durch Anwendung deines Teager Energy Operators (TEO) auszuführen, der definiert ist durch:
TEO {x (n)} = ψ (n) = x² (n-1) -x (n-2) x (n), wobei "x (n)" das erfasste hämodynamische Herzsignal ist, "ψ (n)" das Ausgangssignal ist und "n" sich auf eine vorbestimmte Probe bezieht; und zumindest eine Eigenschaft des Ausgangssignals mit einem vorbestimmten Schwellenwert zu vergleichen; und einen Kammerflimmernalarm auszulösen, wenn die zumindest eine Eigenschaft des Ausgangssignals den vorbestimmten Schwellenwert während eines vorbestimmten Zeitraums nicht überschreitet.

22. Verarbeitungssoftware nach Anspruch 21, die ferner das Vorverarbeiten der erfassten hämodynamischen Herzsignale und/oder der aus den erfassten hämodynamischen Herzsignalen abgeleiteten Signale durch Bandpassfiltration, insbesondere in einem Bereich von 1 bis 100 Hz, ganz insbesondere von 7,5 bis 49 Hz, umfasst.

23. Verarbeitungssoftware nach Anspruch 21 oder 22, die ferner das Nachverarbeiten des Ausgangssignals durch Anwenden einer Fensterfunktion, insbesondere eines Hamming-Fensters, umfasst.

## Claims

1. A medical device, in particular an implantable medical device, comprising: at least one implantable or non-implantable haemodynamic sensor configured to detect cardiac haemodynamic signals;
a controller configured to process and analyse the cardiac haemodynamic signals detected and/or signals derived from cardiac haemodynamic signals detected by applying to said signals, a Teager Energy Operator (TEO) defined by:
TEO {x (n)} = Ψ (n) = x² (n-1) -x (n-2) x (n) where "x (n)" is a cardiac haemodynamic signal, "Ψ (n)" is the output haemodynamic signal and "n" makes reference to a predetermined sample;
said controller further comprising at least one algorithm configured to determine the necessity of a defibrillation operation by considering the at least one output haemodynamic signal.

2. The medical device according to claim 1, wherein the at least one haemodynamic sensor is an implantable or non-implantable accelerometer, a microphone, a piezoelectric sensor or a pressure sensor capable of detecting haemodynamic cardiac signals, in particular cardiac sounds.

3. The medical device according to claim 1 or 2, wherein the at least one algorithm or controller is configured to determine the necessity of a defibrillation operation if at least one characteristic of the output haemodynamic signal does not cross a predetermined threshold during a time lapse which is greater than a duration associated with a ventricular fibrillation episode.

4. The medical device according to claim 3, wherein the at least one characteristic of the output haemodynamic signal is the amplitude, the energy, the mean value or the median value, or the mean square value of said signal.

5. The medical device according to one of claims 3 or 4, wherein the amplitude of the output haemodynamic signals, and/or
the number of times that each of the output haemodynamic signals ascendingly or descendingly crosses the predetermined threshold, and/or
the number of peaks of each signal which exceeds the predetermined threshold during the predefined time lapse,
is/are considered to determine the presence or not of a ventricular fibrillation.

6. The medical device according to one of the preceding claims, further comprising a defibrillator, in particular an implantable defibrillator, configured to generate an electric defibrillation signal; and
at least one electrode connected to said defibrillator by a probe configured to deliver said electric defibrillation signal to a patient when the necessity to trigger a defibrillation operation is determined by means of the controller of the medical device.

7. The medical device according to claim 6, wherein the controller is configured to determine the necessity to trigger a defibrillation operation by comparing the output cardiac haemodynamic signals to at least one electrophysiological signal detected independently to the cardiac haemodynamic signals by means of the at least one electrode.

8. The medical device according to one of claims 1 to 6, wherein the controller is configured to determine the necessity of a defibrillation operation only considering cardiac haemodynamic signals.

9. The medical device according to one of the preceding claims, wherein the controller is configured to filter by bandpass the cardiac haemodynamic signals detected and/or signals derived from the cardiac haemodynamic signals detected in a range of 1 to 100Hz, in particular from 7.5 to 49Hz.

10. The medical device according to one of the preceding claims, wherein the controller is further configured to filter the output signals by application of a window function, in particular a Hamming window.

11. The medical device according to one of claims 3 to 10, wherein the controller is configured to determine the necessity of a defibrillation operation and/or trigger a defibrillation operation according to:
a predefined static threshold, or
according to a dynamic threshold, recalculated periodically and derived from one or
more characteristics of the cardiac haemodynamic signals or of their representation by the Teager Energy Operator.

12. Method for processing cardiac haemodynamic signals detected by at least one haemodynamic sensor of a medical device, in particular an implantable medical device, comprising:
at least one step of processing haemodynamic signals detected and/or signals derived from the cardiac haemodynamic signals detected which comprises the application of a Teager Energy Operator (TEO) defined by:
TEO {x (n)} = Ψ (n) = x² (n-1) -x (n-2) x (n) where "x (n)" is a cardiac haemodynamic signal detected, "Ψ (n)" is an output cardiac haemodynamic signal and "n" makes reference to a predetermined sample;
and further comprising a step during which the presence or not of a ventricular fibrillation is determined by considering the output cardiac haemodynamic signal(s) with respect to a predetermined threshold.

13. The method for processing cardiac haemodynamic signals according to claim 12, of which the step of processing signals is preceded by a step of pre-processing signals in which the haemodynamic signals detected and/or the signals derived from the cardiac haemodynamic signals detected are filter by bandpass, in particular in a range of 1 to 100Hz, more in particular of 7.5 to 49Hz.

14. The method for processing cardiac haemodynamic signals according to claim 12 or 13, of which the step of processing signals is followed by a step of post-processing signals in which the output haemodynamic signals are filtered by application of a window function, in particular by application of a Hamming window.

15. The method for processing cardiac haemodynamic signals according to claim 12, 13 or 14, of which the at least one haemodynamic sensor is an implantable or non-implantable accelerometer with N-axes (N >= 1), a microphone, a piezoelectric sensor or a pressure sensor capable of detecting cardiac haemodynamic signals, in particular cardiac sounds.

16. The method for processing cardiac haemodynamic signals according to claim 15, of which the N cardiac signals detected by the accelerometer with N-axes are combined in a new signal according to an axis N+1 being determined such that the amplitude and/or the signal/sound ratio and/or the stability and/or a relevant physiological parameter of the new signal is maximum according to the axis N+1.

17. The method for processing cardiac haemodynamic signals according to one of claims 12 to 16, of which the predetermined threshold is a predefined static threshold, or a dynamic threshold, recalculated periodically and derived from one or more characteristics of cardiac haemodynamic signals or from their representation by the Teager Energy Operator.

18. The method for processing cardiac haemodynamic signals according to one of claims 12 to 17, wherein the amplitude, and/or
the number of times that each of the output haemodynamic signals ascendingly or descendingly crosses the predetermined threshold, and/or the number of peaks of each signal which exceeds the predetermined threshold during a predefined time lapse,
is/are considered to determine the presence or not of a ventricular fibrillation.

19. The method for processing cardiac haemodynamic signals according to one of claims 12 to 18, wherein the presence or not of a ventricular fibrillation is determined, only considering cardiac haemodynamic signals.

20. The method for processing cardiac haemodynamic signals according to one of claims 12 to 18, wherein the presence or not of a ventricular fibrillation is established by comparing the output haemodynamic signal to at least one electrophysiological signal detected by means of an electrode of the medical device.

21. Software for processing cardiac haemodynamic signals detected by at least one haemodynamic sensor of a medical device, in particular an implantable medical device, **characterised in that** it comprises instructions, when they are executed by the medical device, capable of executing the processing of haemodynamic signals detected and/or signals derived from the cardiac haemodynamic signals detected by application of a Teager Energy Operator (TEO) defined by: TEO {x (n)} = Ψ (n) = x² (n-1) -x (n-2) x (n) where "x (n)" is the cardiac haemodynamic signal detected, "Ψ (n)" is the output signal and "n" makes reference to a predetermined sample; and to compare at least one characteristic of the output signal to a predetermined threshold; and to trigger a ventricular fibrillation alert when the at least one characteristic of the output signal does not cross the predetermined threshold during a predefined time lapse.

22. The processing software according to claim 21, which further comprises the pre-processing of cardiac haemodynamic signals detected and/or signals derived from the cardiac haemodynamic signals detected by bandpass filtration, in particular in a range of 1 to 100Hz, more in particular of 7.5 to 49Hz.

23. The processing software according to claim 21 or 22, which further comprises the post-processing of the output signal by application of a window function, in particular a Hamming window.
